(19) Europäisches Patentamt
European Patent Office
Office européen des brevets

(11) Veröffentlichungsnummer: **0 402 771 B1**

(12) **EUROPÄISCHE PATENTSCHRIFT**

(45) Veröffentlichungstag der Patentschrift: **30.11.94**

(51) Int. Cl.5: **C12P 41/00**, C12P 7/40

(21) Anmeldenummer: **90110794.6**

(22) Anmeldetag: **07.06.90**

# (54) Verfahren zur enzymatischen Spaltung von 2-Arylpropionsäurevinylestern.

(30) Priorität: **10.06.89 DE 3919029**

(43) Veröffentlichungstag der Anmeldung:
**19.12.90 Patentblatt 90/51**

(45) Bekanntmachung des Hinweises auf die Patenterteilung:
**30.11.94 Patentblatt 94/48**

(84) Benannte Vertragsstaaten:
**BE CH DE FR GB IT LI NL**

(56) Entgegenhaltungen:
**EP-A- 0 153 474**
**EP-A- 0 195 717**
**EP-A- 0 299 558**

**TETRAHEDRON LETTERS, Band 28, Nr. 9, 1987, Pergamon Journals Ltd., GB; M. DEGUEIL-CASTAING et al., Seiten 953-954&NUM;**

(73) Patentinhaber: **HOECHST AKTIENGESELLSCHAFT**

**D-65926 Frankfurt (DE)**

(72) Erfinder: **Fülling, Gerd, Dr.**
**Drosselweg 3**
**D-6230 Frankfurt am Main (DE)**
Erfinder: **Schlingmann, Merten, Dr., Prof.**
**Schneidhainer Strasse 32a**
**D-6240 Königstein/Taunus (DE)**
Erfinder: **Keller, Reinold, Dr.**
**Kaunweg 8**
**D-6232 Bad Soden (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 402 771 B1

## Beschreibung

2-Arylpropionsäuren besitzen antiinflammatorische Wirkung. Bei der chemischen Synthese dieser Verbindungen entsteht im allgemeinen das Racemat [J.P. Rieu et al. Tetrahedron Letters 42, 4095 (1986)]. Es ist jedoch bekannt, daß jeweils einer der Enantiomeren eine stärkere biologische Wirkung aufweist. In den meisten Fällen ist dies das S-Enantiomer [E. Hutt, J. Caldwell, Clin. Pharmac. 9 371 (1984)].

J. Iriuchijima et al. [Agric. Biol. Chem. 45 1389 (1981)] beschreiben die mikrobielle Hydrolyse von Naproxen- und Ketoprofen-methylestern, die allerdings bei nur geringem Umsatz stehenbleibt.

In EP 153 474 wird die Racematspaltung von 2-(6-Methoxy-2-naphtyl)-propionsäure-niedrigalkylester beschrieben. Dabei wird in der ersten Stufe des Verfahrens die R-Säure durch Inkubation des Racemats mit Lipasen aus Aspergillus und Bacillus hergestellt und in der zweiten Stufe der zurückgebliebene S-Ester mit unspezifischen Lipasen aus der Schweineleber oder Pleurotus ostreatus hydrolysiert. Hohe Umsätze können nur durch eine kontinuierliche Abtrennung des bei der Spaltung freigesetzten Alkohols erreicht werden, der sich ansonsten auf das Enzym inhibierend auswirken wurde.

Die Europäische Patentanmeldung 195 717 betrifft die Herstellung von S-2-Arylpropionsäure durch Inkubation des racemischen Esters mit einer mikrobiellen Esterase, insbesondere Candida cylindracea. Als Estergruppen werden $-CH_2-C\equiv CH$, $-CH_2-CH=CH_2$, $-CH_2-CN$, $-CH_2-COCH_3$, $-CH_2-COO-(C_1-C_4)$ oder $-CH_2-O-(C_1-C_4)$ eingesetzt. Die Umsatzgeschwindigkeit ist jedoch relativ langsam. Für eine 40 %ige Umsetzung wurden ca. 30 und mehr Stunden benötigt.

Noch wesentlich niedrigere Umsatzgeschwindigkeiten werden in EP 227 078 beschrieben. Zur Herstellung von S-2-Arylpropionsäure werden die racemischen Alkyl-Ester 6 Tage mit mikrobiellen extrazellulären Lipasen behandelt.

EP 233 656 beschreibt ebenfalls die Hydrolyse von $\alpha$-Arylpropionsäureestern in der S-Konfiguration mit Hilfe von neuen bakteriellen Enzymen, die die 10-fache Aktivität von Candida cylindracea besitzen.

In Tetrahedron Letters, Band 28, Nr. 9, 1987, S. 953 und 954 sind enzymatische katalysierte Veresterungen unter Verwendung von Vinylestern beschrieben. Die Umsetzung erfolgt unter wasserfreien Bedingungen und Kochen der Lösemittel unter Rückflußkühlung.

Es wurde nun überraschend gefunden, daß die Umsatzgeschwindigkeiten der Enzyme bei der Hydrolyse von 2-Arylpropionsäureestern, gleichgültig ob sie die Ester der S- oder R-Konfiguration spalten, drastisch erhöht wird, wenn als Substrat der Vinylester der 2-Arylpropionsäure eingesetzt wird.

Die Erfindung betrifft somit ein Verfahren zur Herstellung von optisch aktiven 2-Arylpropionsäurevinylestern durch enzymatische Hydrolyse von 2-Arylpropionsäurvinylester, das dadurch gekennzeichnet ist, daß die Verbindung der allgemeinen Formel I,

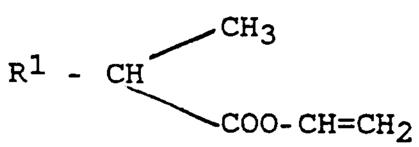

I

in der $R^1$ ein substituierter oder unsubstituierter Arylrest ist, mit Hydrolasen bei einer Temperatur von 10 bis 65 °C und einem pH-Wert von 3 bis 12 inkubiert wird.

Im folgenden wird die Erfindung, insbesondere in ihrer bevorzugten Ausführungsform, detailliert beschrieben. Ferner wird die Erfindung durch den Inhalt der Ansprüche bestimmt.

Als Verbindung der Formel I werden bevorzugt solche eingesetzt, in denen der Rest $R^1$ die Gruppe der Formel II, III oder IV bedeutet,

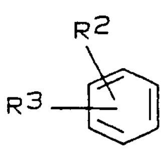

II

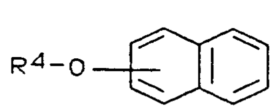

III

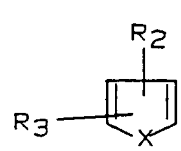

IV

wobei unabhängig voneinander

$R^2$ Wasserstoff, eine verzweigte oder unverzweigte Alkylkette mit 1 bis 8 C-Atomen, Alkenyl mit 2 bis 4 C-Atomen, Alkoxy, Benzoyl, Phenyl, Phenoxy, Thiophencarbonyl, Furancarbonyl oder Pyrrolcar-

bonyl bedeutet,

$R^3$ Wasserstoff oder Halogen,

$R^4$ eine Alkylkette mit 1 bis 4 C-Atomen und

X die Heteroatome S, O oder N bedeutet.

Insbesondere bevorzugt wird in dem erfindungsgemäßen Verfahren die Verbindung der Formel I eingesetzt, in der $R^1$ die Verbindung der Formel II oder III darstellt, in der

$R^2$ Wasserstoff, eine verzweigte oder unverzweigte Alkylkette mit 1 bis 8 C-Atomen, Alkoxy oder Benzoyl ist,

wobei $R^3$ und $R^4$ die obengenannte Bedeutung haben.

Die racemischen 2-Arylpropionsäuren werden nach bekannten Verfahren hergestellt (J. P. Rieu et al. s.o.).

Die Herstellung der Vinylester dieser racemischen 2-Arylpropionsäuren erfolgt auf konventionellem Weg, beispielsweise durch Veresterung mit Vinylacetat in Gegenwart von Palladium- oder Quecksilber-Katalysatoren. (R. Hüttel, Synthesis, 242 (1970)).

Insbesondere bevorzugt werden die Vinylester von racemischer 2-(6-Methoxy-2-naphtyl)-propionsäure (Naproxen®) und 2-(4-Isobutylphenyl)-propionsäure (Ibuprofen®) eingesetzt.

Als Hydrolasen können Lipasen, Esterasen oder Proteasen, die insbesondere mikrobiellen Ursprungs sind, zur Anwendung kommen. Bevorzugt verwendet werden Lipasen oder Esterasen aus Pseudomonas, Mucor, Rhizopus, Penicillium, Geotrichum und insbesondere aus Aspergillus, Candida und Bacillus aber auch Lipasen und Esterasen aus Schweineleber oder Schweinepankreas. Ferner sind Proteasen aus Bacillus, Aspergillus und Rhizopus, insbesondere aus Aspergillus oryzae, bevorzugt.

Die Enzyme sind weitgehend käuflich oder können aus den entsprechenden Quellen nach herkömmlichen Methoden gewonnen werden. Die mikrobiellen Enzyme können beispielsweise nach Kultivierung der Mikroorganismen auf konventionellen Nährmedien nach bekannten Methoden isoliert werden. Es können aber auch die ganzen Mikroorganismen für die erfindungsgemäße Reaktion eingesetzt werden.

Die Enzyme können in freier oder immobilisierter Form zur Anwendung kommen, wobei hierzu alle gängigen Immobilisierungsmethoden in Frage kommen können.

Die Enzymmenge kann in weiten Bereichen schwanken. Sie wird in Abhängigkeit von der Größe des Ansatzes, von der anzustrebenden Reaktionszeit und von der Art des Enzyms gewählt und kann im Einzelfall durch einfache Vorversuche leicht bestimmt werden.

Das zu spaltende Substrat wird u.a. in racemischer Form, d.h. als 1:1-Mischung der S- und R-Enantiomere eingesetzt. Oftmals kann aber die optische Ausbeute dadurch erhöht werden, daß man bereits optisch angereichertes Substrat, hervorgegangen beispielsweise aus der enzymatischen Hydrolyse, Kristallisation o.ä., zur enzymatischen Spaltung einsetzt.

Zur Spaltung arbeitet man in Suspension, wobei ein Substrat-Puffer-Verhältnis von 0,1 Gew.-% bis 30 Gew.-% eingestellt werden kann, bevorzugt wird 1 bis 10 Gew.-%.

Die Reaktion wird bei 10 bis 65 °C, bevorzugt 20 bis 50 °C durchgeführt, wobei natürlich die Abhängigkeit der Aktivität des jeweiligen Enzyms von der Temperatur zu berücksichtigen ist. Der pH-Wert der Reaktionslösung liegt ebenfalls entsprechend der Aktivtät des Enzyms im Bereich von pH 3 bis 12, bevorzugt 5 bis 9, insbesondere zwischen 6 und 8,5. Das Enzym/Substrat-Verhältnis kann in Abhängigkeit von der geforderten Reaktionsgeschwindigkeit im Bereich von 0,05 Gew.-% bis 100 Gew.-% liegen, bevorzugt aber zwischen 1 und 20 Gew.-%. Im kontinuierlichen Säulenverfahren kann das durch die Konzentration der Substratlösung bedingte lokale Enzym/Substrat-Verhältnis sogar noch 100 Gew.-% übersteigen.

Nach der hydrolytischen Spaltung liegen die optischen Antipoden als Carbonsäure bzw. als Vinylester vor und können entsprechend ihres unterschiedlichen physikalischen bzw. chemischen Verhaltens durch Destillation, Kristallisation, Chromatographie oder andere gängige Verfahren getrennt werden, bevorzugt aber durch Extraktion derart, daß bei alkalischem pH der Ester mit einem geeigneten organischen Lösungsmittel, wie beispielsweise Chloroform, Methylenchlorid, tert.-Butylmethylether, Methylisobutylketon etc. erschöpfend extrahiert wird, wobei die Säure zunächst als Alkalisalz in der wäßrigen Phase verbleibt. Anschließend kann man die 2-Arylpropionsäure als amorphen Niederschlag bei niedrigem pH-Wert (pH 1 bis 4) ausfällen und abzentrifugieren oder extraktiv mit obengenannten Lösungsmitteln entfernen.

Zur Erhöhung der optischen Ausbeute kann der gespaltene, optisch angereicherte Vinylester in einer wiederholten Spaltung gemäß dem oben beschriebenen Prozeß mit dem gleichen oder einem anderen, entgegengesetzte Stereoselektivität aufweisenden Enzym umgesetzt werden.

Zur Freisetzung der freien Carbonsäuren aus den optisch aktiven Vinylestern kann man wahlweise eine säurekatalysierte Hydrolyse oder eine palladiumsalzkatalysierte Transvinylierung in Eisessig durchführen.

Die Darstellung optisch aktiver Vinylester aus den korrespondierenden 2-Arylpropionsäuren gelingt, überraschenderweise unter Erhalt der vollen optischen Aktivität, durch palladiumkatalysierte Transvinylierung in Vinylacetat. Als Katalysator wird bevorzugt $Li_2PdCl_4$ auf Aktivkohle als Trägermaterial eingesetzt. Die Transvinylierung wird bevorzugt bei Siedetemperatur des Reaktionsgemisches durchgeführt.

Die 2-Arylpropionsäurevinylester weisen eine gute antiinflammatorische Wirkung auf.

In den folgenden Beispielen wird die Erfindung weitergehend erläutert. Die Prozentangaben beziehen sich auf das Gewicht, wenn nichts anderes angegeben.

**Beispiel 1**

**Darstellung der Vinylester**

a) 100 g 2-(4'-Isobutylphenyl)-propionsäure (Ibuprofen®) wurden in 1 l Vinylacetat aufgenommen und in Gegenwart von 2 g $Li_2PdCl_4$ und 20 g Aktivkohle unter Rückfluß zum Sieden erhitzt. Die Reaktion wurde dünnschichtchromatographisch verfolgt. Nach 8 Stunden filtrierte man den Katalysator ab und engte das Filtrat zur Trockne ein. Nach Filtration über Kieselgel mit Hexan:Essigester (10:1) erhielt man 92,7 g (39,9 mmol; 82 %) 2-(4'-Isobutylphenyl)-propionsäurevinylester als klares Öl.

b) 100 g (0,43 mol) 2-(6-Methoxy-2-naphthyl)-propionsäure wurden analog Beispiel 1 in den Vinylester übergeführt. Ausbeute: 87 g (78 %)

Schmp.: 69 °C.

**Beispiel 2**

**Enzymatische Esterspaltung**

10 g (4,3 mmol) 2-(4'-Isobutylphenyl)-propionsäurevinylester wurden in 200 ml 0,1 M Phosphatpuffer (pH 7,0) suspendiert und nach Zugabe von 2 g Protease aus Aspergillus oryzae (Sigma Typ XXIII) gerührt.

Der pH-Wert wurde durch Zugabe von 0,5 M NaOH konstant gehalten. Nach 24 Stunden wurde die Reaktion bei einem Umsatz von 53 % abgebrochen. Bei pH 8 wurde dann der verbleibende S-Ester mit Methylenchlorid oder Methylisobutylketon extrahiert und die organische Phase zur Trockne eingeengt.

a. Ausbeute: 4,0 g (40 %) S-2-(4'-Isobutylphenyl)-propionsäurevinylester

$[\alpha]_D^{20}$ = +7 (c = 1, $CHCl_3$)

ee ≧ 98 % (Enantionemerenüberschuß: Bestimmung über $^1H$-NMR nach Zugabe von Shiftreagenz Eu $(hfc)_3$ ≙ Tris[3-(heptafluoropropylhydroxymethylen)-(+)-camphorato], europium (III) derivat)

b. Ausbeute: 3,1 g (35 % ) R-2-(4'-Isobutylphenyl)-propionsäure $[\alpha]_D^{20}$ = -49 (c = 1, $CHCl_3$)

ee: 85 % (Bestimmung über $^1H$-NMR durch Überführung der Säure in den Methylester mit Diazomethan und Vermessung des Methylesters nach Zugabe von Shiftreagenz Eu $(hfc)_3$)

**Beispiele 3-7**

Jeweils 200 mg (0,86 mmol) 2-(4'-Isobutylphenyl)-propionsäurevinylester wurden mit jeweils 50 mg Enzym in 5 ml 0,5 M Phosphatpuffer (pH 7,8) bei Raumtemperatur intensiv gerührt. Nach beendeter Reaktion wurden der verbleibende 2-(4'-Isobutylphenyl)-propionsäurevinylester und 2-(4'-Isobutylphenyl)-propionsäure, wie unter Beispiel 2 beschrieben, getrennt. Die Ergebnisse sind in Tab. 1 zusammengefaßt.

| Beispiel | Enzym | Reaktionszeit [h] | Umsatz [%] | Substrat | | Produkt | | |
|---|---|---|---|---|---|---|---|---|
| | | | | ee [%] [a)] | Konfiguration | $[\alpha]^{20}_{D}$ [b)] | ee [%] [a)] | Konfiguration |
| 3 | M-AP [c)d)] | 24 | 67 | 54 | S | -15 | 27 | R |
| 4 | F-AP [d)e)] | 5 | 38 | 18 | S | -16 | 29 | R |
| 5 | Typ VII [f) h)] | 28 | 49 | 65 | S | -39 | 67 | R |
| 6 | Typ XIX [f)g)] | 71 | 44 | 67 | S | -48 | 86 | R |
| 7 | F 7 [n)] | 4 | 87 | 98 | R | + 9 | 16 | S |

a) bestimmt wie unter Beispiel 2 beschrieben; b) c = 1, $CHCl_3$; c) Lipase aus Mucor javanicus, d) von Amano Pharmaceutical Co., LTD; e) Lipase aus Rhizopus javanicus; f) von Sigma Chemie GmbH; g) Protease aus Aspergillus sojae; h) Lipase aus Candida cylindracea; n) Lipasen von Enzymatix LTD

**Beispiele 8-20**

Jeweils 200 mg (0,78 mmol) 2-(6-Methoxy-2-naphthyl)-propionsäurevinylester wurden mit 50 mg Enzym in 5 ml 0,5 M Phosphatpuffer bei Raumtemperatur intensiv gerührt. Nach beendeter Reaktion wurden 2-(6-Methoxy-2-naphthyl)-propionsäurevinylester und 2-(6-Methoxy-2-naphthyl)-propionsäure, wie unter Beispiel 2 beschrieben, getrennt. Die Ergebnisse sind in Tab. 2 zusammengefaßt.

| Beispiel | Enzym | pH | Reaktions-zeit [h] | Umsatz [%] | Substrat | | Produkt | | |
|---|---|---|---|---|---|---|---|---|---|
| | | | | | ee [%][a)] | Konfiguration | $[\alpha]^{20}_{D}$ [b)] | ee [%][a)] | Konfiguration |
| 8 | M-AP[c)d)] | 7,0 | 72 | 62 | 90 | S | n.b. | 55 | R |
| 9 | F-AP[d)e)] | 7,0 | 96 | 42 | 56 | S | -49 | 77 | R |
| 10 | Typ XIX[f)g)] | 7,8 | 48 | 30 | 35 | S | n.b. | 80 | R |
| 11 | Typ XXIII[f)o)] | 7,8 | 96 | 44 | 75 | S | n.b. | 95 | R |
| 12 | OF[i)k)] | 7,0 | 8 | 57 | 44 | R | n.b. | 30 | S |
| 13 | Protease N[d)l)] | 7,8 | n.b. | 13 | 10 | S | -46 | 70 | R |
| 14 | Subtilisin[m)] | 7,8 | n.b. | 53 | 52 | R | +30 | 46 | S |
| 15 | F1[n)] | 7,8 | 91 | 43 | 70 | S | -62 | 94 | R |
| 16 | F3[n)] | 7,8 | 24 | 45 | 74 | S | -59 | 90 | R |
| 17 | F5[n)] | 7,8 | 24 | 80 | 80 | R | +13 | 20 | S |
| 19 | F7[n)] | 7,8 | 24 | 68 | 78 | S | -19 | 37 | R |
| 19 | F8[n)] | 7,8 | 48 | 56 | 86 | S | -45 | 68 | R |
| 20 | PLE[f)] | 7,0 | 48 | 13 | 4 | S | -17 | 26 | R |

**Fortsetzung Tab. 2:**

n.b. ≡ nicht bestimmt

a)-g), n) siehe entsprechende Fußnoten Tab. 1

h) Protease aus Aspergillus oryzae

i) Lipase aus Candida cylindracea

k) Meito Sangyo Co LTD

l) Protease aus Bacillus sp.

m) Novo Industri

o) Protease aus Aspergillus oryzae

**Beispiel 21**

10 g (43 mmol) racemischer 2-(4'-Isobutylphenyl)-propionsäurevinylester wurden in 200 ml 0,1 M Phosphatpuffer (pH 7,0) in Gegenwart von 200 mg Lipase OF (aus Candida cylindracea, Meito Sangyo LTD) 7 h analog Beispiel 2 umgesetzt. Man isolierte:

a) 4,3 g (43 % R-2-(4'-Isobutylphenyl)-propionsäurevinylester
$[\alpha]_D^{20}$ = -5 (c = 1, $CHCl_3$)
ee = 76 %

b) 4,2 g (47 %) S-2-(4'-Isobutylphenyl)propionsäure
$[\alpha]_D^{20}$ = +37 (c = 1, $CHCl_3$)
ee = 68 %

**Beispiel 22**

1 g (4,8 mmol) S-2-(4'-Isobutylphenyl)-propionsäure (ee = 68 %) aus Beispiel 21 wurde in 10 ml Vinylacetat in Gegenwart von 125 mg $Li_2PdCl_4$ und 1,25 g Aktivkohle analog Beispiel 1 umgesetzt.
Ausbeute: 900 mg (81 %) S-2-(4'-Isobutylphenyl)-propionsäurevinylester
$[\alpha]_D^{20}$ = +5 (c = 1, $CHCl_3$)
ee = 68 %

**Beispiel 23**

680 mg (2,9 mmol)
S-2-(4'-Isobutylphenyl)-propionsäurevinylester (ee = 68 %) aus Beispiel 22 wurden in 50 ml 0,1 N Phosphatpuffer (pH 7,0) suspendiert und nach Zugabe von 50 mg Lipase OF (aus Candida cylindracea, Meito Sangyo LTD) bei Raumtemperatur unter pH-Konstanz (durch Zudosieren von 0,1 N NaOH) 5 h gerührt. Anschließend stellte man mit NaOH auf pH 10, wusch mit Methylenchlorid den nicht umgesetzten Vinylester aus, stellte die wässrige Phase dann auf pH 2 und extrahierte, die S-2-(4'-Isobutylphenyl)-propionsäure mit Methylenchlorid. Die organische Phase wurde zur Trockne eingeengt und anschließend das gewünschte Produkt einmal aus Hexan umkristallisiert.
Ausbeute: 305 mg (51 %) S-2-(4'-Isobutylphenyl)-propionsäure
$[\alpha]_D^{20}$ = +54 (c = 1, $CHCl_3$)
ee = 96 %.

**Beispiel 24**

**Spaltung des S-Esters**

2,8 g (12,1 mmol) S-Ibuprofenvinylester aus Beispiel 2 wurden in 60 ml Eisessig aufgenommen und in Gegenwart von 200 mg $Li_2PdCl_4$ auf Aktivkohle bei 60-70 °C gerührt. Nach Beendigung der Reaktion (DC-Kontrolle) filtrierte man den Katalysator ab und engte das Filtrat zur Trockne ein. Man nahm in Methylisobutylketon auf, wusch einmal mit Wasser und engte zur Trockne ein. Man kristallisierte einmal aus Hexan um.
Ausbeute: 2,4 g Ibuprofen (11,6 mmol) 96 %
$[\alpha]_D^{20}$ = +57 (c = 1, $CHCl_3$)
ee: ≧ 98 %.

**Beispiel 25**

Jeweils 100 mg 2-(4'-Isobutylphenyl)-propionsäureester ([a]Methyl, [b]Chlorethyl, [c]Vinyl) wurden in 40 ml 0,25 M Phosphatpuffer (pH 7,8) suspendiert und nach Zugabe von 100 mg Protease aus Aspergillus oryzae (Sigma) bei 35 °C gegen 0,1 N NaOH titriert. Der Umsatz wurde über die zudosierte NaOH berechnet. Abb. 1 zeigt die Zeit-Umsatz-Abhängigkeit der enzymatischen Hydrolyse des jeweiligen 2-(4'-Isobutylphenyl)-propionsäureester.

## Patentansprüche

1. Verfahren zur Herstellung von optisch aktiven 2-Arylpropionsäurevinylestern durch enzymatische Hydrolyse von racemischen 2-Arylpropionsäurevinylester, dadurch gekennzeichnet, daß die Verbindung der allgemeinen Formel I,

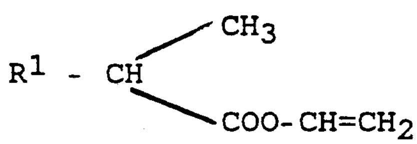

I,

in der $R^1$ ein substituierter oder unsubstituierter Arylrest ist, mit Hydrolasen bei einer Temperatur von 10 bis 65 °C und einem pH-Wert von 3 bis 12 inkubiert wird.

2. Verfahren nach Anspruch 1, dadurch gekennzeichnet, daß der Rest $R^1$ die Gruppe der Formel II, III oder IV bedeutet,

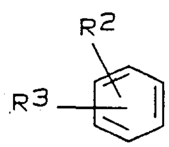

II

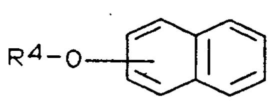

III

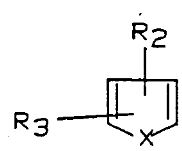

IV

in denen unabhängig voneinander

- $R^2$ Wasserstoff, eine verzweigte oder unverzweigte Alkylkette mit 1 bis 8 C-Atomen, Alkenyl mit 2 bis 4 C-Atomen, Alkoxy, Benzoyl, Phenyl, Phenoxy, Thiophencarbonyl, Furancarbonyl oder Pyrrolcarbonyl ist,
- $R^3$ Wasserstoff oder Halogen,
- $R^4$ eine Alkylkette mit 1 bis 4 C-Atomen und
- X die Heteroatome s, O oder N bedeutet.

3. Verfahren nach Anspruch 1 oder 2, dadurch gekennzeichnet, daß als Hydrolasen Lipasen, Esterasen oder Proteasen verwendet werden.

4. Verfahren nach Anspruch 3, dadurch gekennzeichnet, daß Lipasen oder Esterasen aus Aspergillus, Bacillus, Candida, Mucor, Rhizopus, Penicillium, Geotrichum, Pseudomonas sowie Schweineleber und Schweinepankreas eingesetzt werden.

5. Verfahren nach Anspruch 3, dadurch gekennzeichnet, daß Proteasen aus Bacillus, Aspergillus und Rhizopus eingesetzt werden.

6. Verfahren nach Anspruch 1, dadurch gekennzeichnet, daß die Reaktion bei 20 bis 50 °C durchgeführt wird.

7. Verfahren nach Anspruch 1, dadurch gekennzeichnet, daß die Reaktion bei einem pH-Wert von 5 bis 9 durchgeführt wird.

8. Optisch aktive Arylpropionsäurevinylester der allgemeinen Formel I

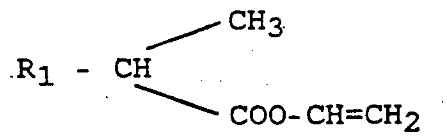

I

in der $R_1$ die Gruppe der Formel II, III oder IV bedeutet,

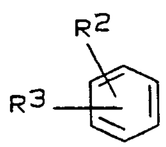

II

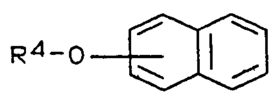

III

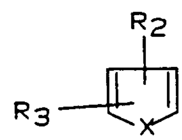

IV

in denen unabhängig voneinander

$R^2$ Wasserstoff, eine verzweigte oder unverzweigte Alkylkette mit 1 bis 8 C-Atomen, Alkenyl mit 2 bis 4 C-Atomen, Alkoxy, Benzoyl, Phenyl, Phenoxy, Thiophencarbonyl, Furancarbonyl oder Pyrrolcarbonyl ist,

$R^3$ Wasserstoff oder Halogen,

$R^4$ eine Alkylkette mit 1 bis 4 C-Atomen ist und

X die Heteroatome S, O oder N bedeutet.

**Claims**

1. A process for the preparation of optically active vinyl 2-arylpropionates by enzymatic hydrolysis of racemic vinyl 2-arylpropionates, which comprises incubating the compound of the formula I

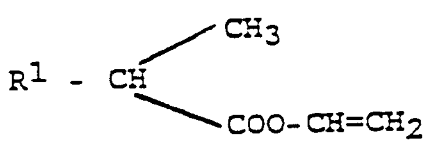

I

in which $R^1$ is a substituted or unsubstituted aryl radical, with hydrolases, at a temperature of 10 to 65 °C and a pH of 3 to 12.

**2.** The process as claimed in claim 1, wherein the radical $R^1$ is the group of the formula II, III or IV

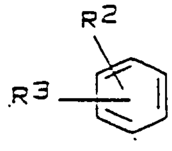
II
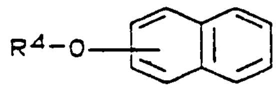
III
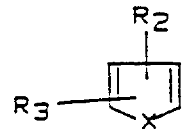
IV

in which, independently of one another,

- $R^2$ is hydrogen, a branched or unbranched alkyl chain having 1 to 8 carbon atoms, alkenyl having 2 to 4 carbon atoms, alkoxy, benzoyl, phenyl, phenoxy, thiophenecarbonyl, furancarbonyl or pyrrolecarbonyl,
- $R^3$ is hydrogen or halogen,
- $R^4$ is an alkyl chain having 1 to 4 carbon atoms, and
- X is a heteroatom S, O or N.

**3.** The process as claimed in claim 1 or 2, wherein lipases, esterases or proteases are used as hydrolases.

**4.** The process as claimed in claim 3, wherein lipases or esterases from Aspergillus, Bacillus, Candida, Mucor, Rhizopus, Penicillium, Geotrichum, Pseudomonas and pig liver and pig pancreas are employed.

**5.** The process as claimed in claim 3, wherein proteases from Bacillus, Aspergillus and Rhizopus are employed.

**6.** The process as claimed in claim 1, wherein the reaction is carried out at 20 to 50 °C.

**7.** The process as claimed in claim 1, wherein the reaction is carried out at a pH of 5 to 9.

**8.** An optically active vinyl arylpropionate of the formula I

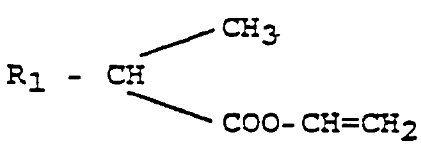
I

in which $R^1$ is the group of the formula II, III or IV

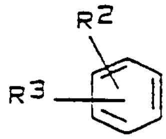
II
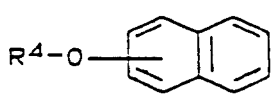
III
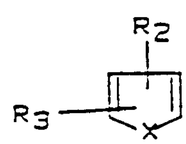
IV

in which, independently of one another,

- $R^2$ is hydrogen, a branched or unbranched alkyl chain having 1 to 8 carbon atoms, alkenyl having 2 to 4 carbon atoms, alkoxy, benzoyl, phenyl, phenoxy, thiophenecarbonyl, furancarbonyl or pyrrolecarbonyl,
- $R^3$ is hydrogen or halogen,
- $R^4$ is an alkyl chain having 1 to 4 carbon atoms and
- X is a heteroatom S, O or N.

## Revendications

1. Procédé pour la préparation d'esters vinyliques d'acides 2-arylpropioniques optiquement actifs, par hydrolyse enzymatique d'esters vinyliques d'acides 2-arylpropioniques racémiques, caractérisé en ce que le composé de formule générale I

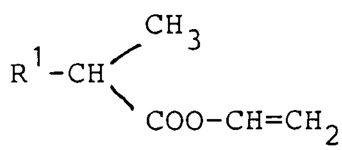

I

dans laquelle $R^1$ est un radical aryle substitué ou non substitué, est mis à incuber avec des hydrolases, à une température de 10 à 65 °C et à un pH de 3 à 12.

2. Procédé selon la revendication 1, caractérisé en ce que le radical $R^1$ représente un groupe de formule II, III ou IV,

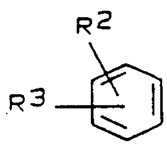

II

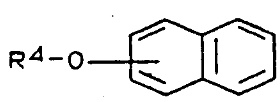

III

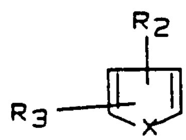

IV

formules dans lesquelles, indépendamment les uns des autres,

- $R^2$ représente un atome d'hydrogène ou une chaîne alkyle ramifiée ou non ramifiée, ayant de 1 à 8 atomes de carbone, un radical alcényle ayant de 2 à 4 atomes de carbone, alcoxy, benzoyle, phényle, phénoxy, thiophène-carbonyle, furannecarbonyle ou pyrrolcarbonyle,
- $R^3$ représente un atome d'hydrogène ou d'halogène,
- $R^4$ représente une chaîne alkyle ayant de 1 à 4 atomes de carbone, et
- X représente les hétéroatomes S, O ou N.

3. Procédé selon la revendication 1 ou 2, caractérisé en ce que, en tant qu'hydrolases, on utilise des lipases, des estérases ou des protéases.

4. Procédé selon la revendication 3, caractérisé en ce que l'on utilise des lipases ou des estérases provenant d'Aspergillus, Bacillus, Candida, Mucor, Rhizopus, Penicillium, Geotrichum, Pseudomonas ainsi que du foie de porc et du pancréas de porc.

5. Procédé selon la revendication 3, caractérisé en ce que l'on utilise des protéases de Bacillus, Aspergillus et Rhizopus.

6. Procédé selon la revendication 1, caractérisé en ce que la réaction est effectuée à 20-50 °C.

7. Procédé selon la revendication 1, caractérisé en ce que la réaction est effectuée à un pH de 5 à 9.

8. Esters vinyliques d'acides arylpropioniques optiquement actifs de formule générale I

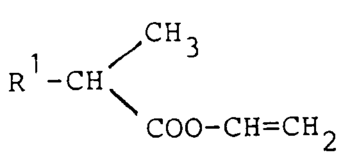

I

dans laquelle $R^1$ représente un groupe de formule II, III ou IV,

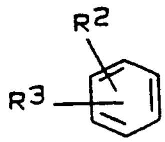

II

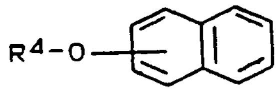

III

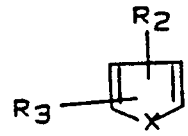

IV

formules dans lesquelles, indépendamment les uns des autres,

- $R^2$ représente un atome d'hydrogène ou une chaîne alkyle ramifiée ou non ramifiée, ayant de 1 à 8 atomes de carbone, un radical alcényle ayant de 2 à 4 atomes de carbone, alcoxy, benzoyle, phényle, phénoxy, thiophène-carbonyle, furannecarbonyle ou pyrrolcarbonyle,
- $R^3$ représente un atome d'hydrogène ou d'halogène,
- $R^4$ représente une chaîne alkyle ayant de 1 à 4 atomes de carbone, et
- X représente les hétéroatomes S, O ou N.

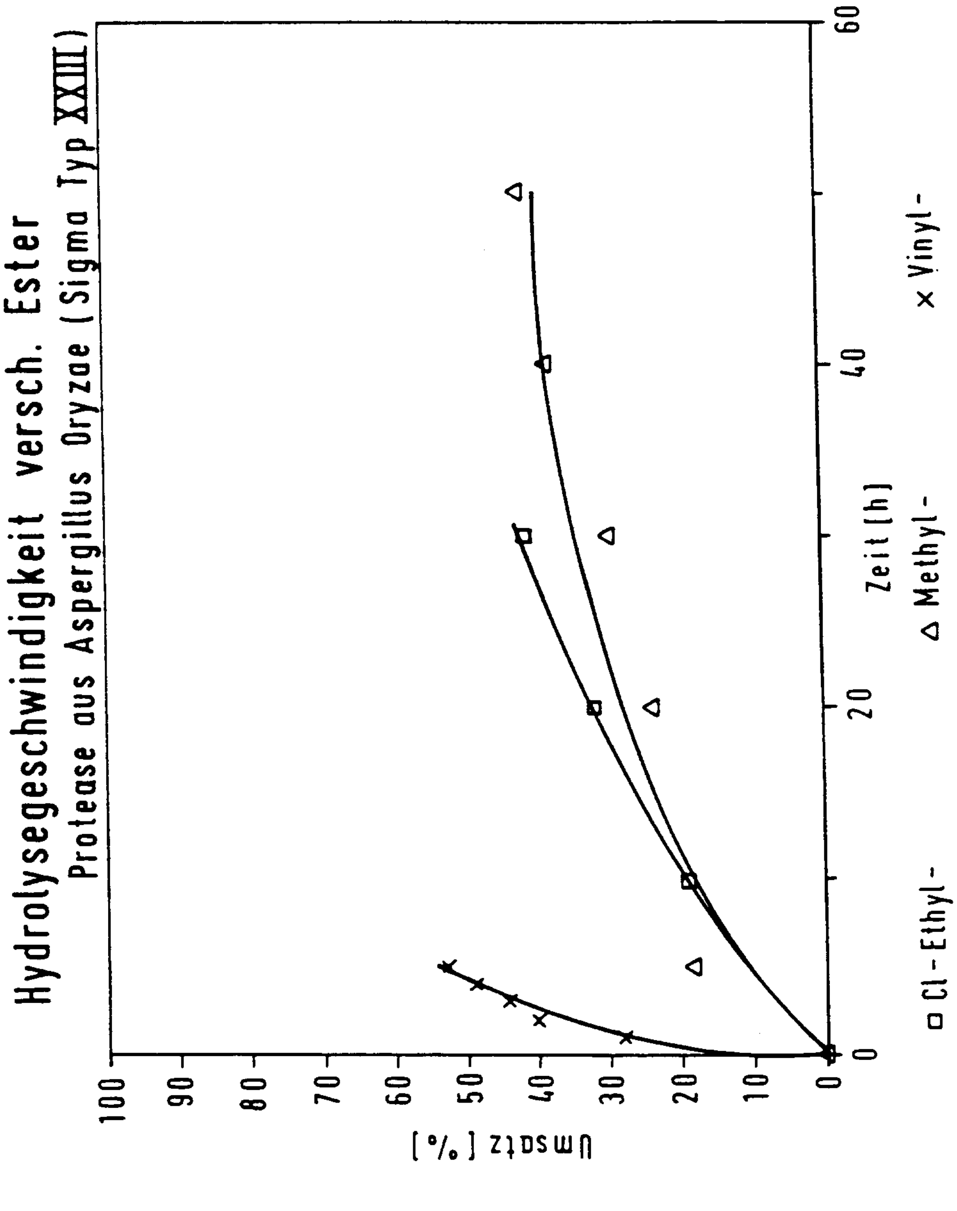
Hydrolysegeschwindigkeit versch. Ester
Protease aus Aspergillus Oryzae (Sigma Typ XXIII)
Umsatz [%]
100
90
80
70
60
50
40
30
20
10
0
0
20
40
60
Zeit [h]
□ Cl-Ethyl-
△ Methyl-
× Vinyl-